# EUROPEAN PATENT SPECIFICATION

(11) **EP 0 798 386 B1**
(45) Date of publication and mention of the grant of the patent: **06.05.2004**
(21) Application number: 97500058.9
(22) Date of filing: 25.03.1997
(51) Int. Cl.: C12Q 1/04, C12N 5/10, G01N 33/533

(54) **Procedure for the analysis of the cell cycle of cell subpopulations present in heterogeneous cell samples**
Verfahren zur Analyse vom Zellzyklus von Zellsubpopulationen aus heterogenen Zellproben
Procédé d'analyse du cycle cellulaire de sous-populations de cellules présentes dans des échantillons de cellules hétérogénes

(30) Priority: 26.03.1996 ES 9600715
(43) Date of publication of application: 01.10.1997
(73) Proprietor: UNIVERSIDAD DE SALAMANCA, 37007 Salamanca (ES)
(72) Inventor: Orfao de Matos Correia e Vale, José Alberto, 37194 Santa Maria de Tormes, Salamanca (ES)
(74) Representative: Hernandez Covarrubias, Arturo

(56) References cited:
- EP-A- 0 121 262
- US-A- 4 780 406
- CELL TISSUE KINET 22 (3). 1989. 223-234. CODEN: CTKIAR ISSN: 0008-8730, XP002035696 MCMURRAY B P ET AL: "SEQUENTIAL FLOW CYTOMETRIC ANALYSIS OF CELL - CYCLE RELATED CHANGES IN LFA-1 CD18-CD11A EXPRESSION BY TRISOMY 21 DOWN'S SYNDROME LYMPHOBLASTOID CELLS."
- BLOOD 67 (3). 1986. 676-681. CODEN: BLOOAW ISSN: 0006-4971, XP002035697 ANDREEFF M ET AL: "CELLULAR RAS ONCOGENE EXPRESSION AND CELL CYCLE MEASURED BY FLOW CYTOMETRY IN HEMATOPOIETIC CELL LINES."

## Description

This invention deals mainly, although not exclusively, with a procedure for the rapid cost-effective, sensitive and specific flow cytometric analysis of the cell cycle of tumor and normal cell subpopulations present in heterogeneous cell samples, in order to increse the knowledge of the cell biology for diagnostic, prognostic and therapeutic purposes and research.

Cell cycle applies to the phases through which a cell has to pass in order to multiplicate itself and give rise to two theoretically identical daughter cells. During this period and in a sequential way, resting cells (G0-phase) will, under certain circumstances or after receiving specific stimuli, initiate the synthesis of RNA and proteins (G1-phase) which are necessary to effectively carry out the multiplication of its DNA and the division of the cell into two daughter cells. Subsequentially, DNA synthesis begins (S-phase); once the cell has duplicated its DNA, a second late-protein-synthesis period begins (G2-phase) which prepares the cell for division (M-phase). Cell cycle analysis, mainly through the study of the distribution of cells throughout the G0/G1, S and G2-M cell cycle phases has proven to be of use in the analysis of tumor samples and the study of the proliferative response to different stimuli as well as in other areas. Overall, the method is of enormous value, not only to gain insight in the area of cell biology but also for diagnostic, prognostic and therapeutic purposes. Of the different methodologies used today the most extensively employed is based on the staining of cells with DNA specific fluorochromes. From the amount of fluorescence/DNA obtained and using mathematical models, the proportion of cells from a certain sample which are in the G0/G1, S and G2/M cell cycle phases is calculated. One of the major pitfalls for the analysis of the cell cycle in samples obtained in vivo is the frequent absence of pure cell populations; Frequently (i.e. in tumor samples or other biological samples) a mixture of different cell types exists at variable proportions which may present a different distribution along the distinct cell cycle phases. Therefore, the global analysis of the cell cycle does not provide direct information on the proliferation of each of the different subpopulations present in the sample. Recent studies have shown the possibility of performing staining with monoclonal antibodies and a DNA fluorochrome in order to specifically analyze the distribution of tumor cells along the different cell cycle phases (Dolbeare Frank A et al; cell tissue Kinet 22(3)). However vvarious factors make it more difficult to differentiate the cells for specific identification from the remaining cells in order to analyze their cell cycle distribution. These factors include: the heterogeneity of the tumor cells regarding expression of an individual marker (either within the same tumor sample or between different tumor samples from different patients with the same diagnosis), the use of fluorochromes that bind specifically to the DNA which absorb part of the fluorescence associated with the monoclonal antibody used and the necessity of permeabilizing substancies in most of these procedures which to a certain extent alter the cell membrane and the binding of the monoclonal antibody. (Becton Dickinson Co)

Accordingly, until now, no procedure has been reported in which, within a sample where the cell cycle distribution is to be analyzed using staining with fluorochromes specific for nucleic acids the same fluorocchrome is used on a pool of different monoclonal antibodies to identify a cell population.

Therefore one aim of this invention is to offer a solution for the specific study of the cell cycle for a population of cells present in a sample in which an optimal discrimination between the cells of interest and the remaining cell elements of the sample may be obtained.

In addition, another aim of the invention is to determine the distribution of the remaining cells (cells which ore negative for the pool of monoclonal antibodies used) throughout the different cell cycle phases.

The procedure for this invention consists in using a pool of monoclonal antibodies conjugated with the some fluorochrome (FL1), such as fluorescein isothiocyanate (FITC), to identify all cells present in the sample to be specifically identified, and a fluorochrome specific for nucleic acids (FL2), such as propidium iodide or ethidium bromide.

Sample preparation, cell staining with monoclonal antibodies and nucleic acid specific fluorochromes, the selection of the cells to be studied as well as the calibration and adjustment of the flow cytometer, are performed following previously described and recommended methods (Becton Dickinson Co).

For the analysis of the results as well as for the exact quantitation of the distribution of each cell population studied in each cell cycle phase, software programs which ore commercially available can be used. During the analysis, besides selecting the subgroup of cells of interest for the study from the sample, any events that correspond to cell multiplets as well as cell debris must be excluded.

The invention can be used both in normal and pathological samples, from human or experimental animal models, from vegetables, bacteria and other microorganisms, and for all purposes where an analysis is required of the distribution of a subgroup of cells present in a sample along the G0/G1, S and G2/M cell cycle phases, for research and diagnostic prognostic and therapeutic studies.

As may be deduced from what has been described above, the monoclonal antibodies used can vary, basically depending on the cell type to be identified. Moreover, it should be understood that in this invention, variations in which a different type of fluorochrome is used, or in which the number of monoclonal antibodies is higher than two are included. Finally, any variations in which more than one pool of monoclonal antibodies are used, in which they are stained with different fluorochromes for specific and simultaneous identification in the same sample of two or more different cell subpopulations, are also included.

With this invention we optimize, in a significant way, the study of the cell cycle in biologic samples and we improve the selection of cells of interest as well as the speed of their study.

It should also be pointed out that the possibility of using a pool of monoclonal antibodies stained with the same fluorochrome for the study of the cell cycle provides detailed information on the proliferation of the cells studied which would help to systematically identify their location and role in different normal and pathological conditions.

Without setting a limit on the invention's potential, two examples are described below, which illustrate its use.

### EXAMPLE 1

### 1.-Material and methods.

Peripheral blood (PB) was obtained by venous puncture from 10 patients diagnosed as suffering from B-cell chronic lymphoproliferative disorders, placed in liquid EDTA (K3) and mantained at room temperature until sample preparation was begun. Cell cycle analysis was performed within the next 5 hours using a direct immunofluorescence technique and staining with propidium iodide measured at flow cytometry.

### 2.-Sample preparation.

A tube containing 100 µL of PB with 0.5-1 x 10⁶ white blood cells was prepared for each of the samples. The sample was incubated with four monoclonal antibodies conjugated with FITC.

The specificity of the combinations of the monoclonal antibodies used and its origin were as follows:
1) FMC63-FITC (CD19); pan-B marker (Serotec,UK)
2) Leu 14-FITC (CD22); pon-B marker (Becton/Dickinson, USA)
3) Leu16-FITC (CD20); pan-B marker (Becton/Dickinson, USA)
4) WR17 -FITC (CD37); pan-B marker (Serotec, UK)

The tubes were gently vortexed and incubated in the dark for 10-15 minutes at room temperature. Immediately after this incubation period, 2 mL of an amonium chloride lysing solution was added, followed by a 4-5 second vortex. Afterwards, cells were incubated for another . 10 minutes in the dark (room temperature). Afterwards samples were centrifuged at 300g for 5 minutes (4°C). The supernnatant was discarded and the cell pellet free of red blood cells was resuspended by vigorous vortexing (1-2 seconds). In each tube 1 mL of a phosphate buffer saline solution (PBS) containing 1 g/L of bovine serum albumine (Sigma Chemicals, St Louis, MO, USA) was added. Another centrifugation (300g, 5 minutes), was performed and cells were finally resuspended in 1 mL of PBS solution containing 0.5% tween-20 (Sigma), 100mg/mL de RNAse (Sigma Chemicals) and 5µg/mL of propidium iodide (Sigma Chemicals). Cells were incubated for 15 minutes at room temperature in this solution and were stored at 4°C in the darkness until analyzed in the flow cytometer.

### 3.- Data acquisition and analysis.

Measurements were performed on a FACSort (Becton/Dickinson) flow cytometer equipped with on argon ion laser tuned at 488nm and 15 m watts. The instrument was calibrated using the DNA-QC reagent (Becton/Dickinson). Fluorescence compensation between FITC and propidium iodide was performed using CALIBRITE beads (Becton/Dickinson) and chicken red cells stained with propidium iodide.

Data analysis was performed in two steps. First, we selected the cells of interest (FITC positive) corresponding to neoplastic B-cells and excluding not only non-B cells but also cell debris and cell aggregates. For that purpose the PAINT-A-GATE PLUS (Becton/Dickinson) software program was used. Afterwards the RFIT mathematical model included in the CELLFIT software program (Becton/Dickinson) was used for the analysis of DNA histograms in order to calculate the proportion of cells from the selected cell population present in the G0/G1, S y G2/M cell cycle phases.

### EXAMPLE 2

### 1.-Material and methods.

Peripheral blood (PB) was obtained by venous puncture from 15 patients diagnosed as suffering from multiple myeloma, placed in liquid EDTA (K3) and mantained at room temperature until sample preparation was begun. Cell cycle analysis was performed within the next 5 hours using an indirect immunofluorescence technique and staining with propidium iodide measured at flow cytometry.

### 2.-Sample preparation.

A tube containing 100 µL of PB with 0.5-1 1 x 10⁶ white blood cells was prepared for each of the samples. The sample was incubated with two monoclonal antibodies.

The specificity of the combinations of the monoclonal antibodies used and their origin were as follows:
1) BB4; Plasma cell marker (Serotec, UK)
2) GR7A4 (CD38); Marker that is strongly and characteristically expressed by plasma cells (Universidad de Granada, Spain)

The tubes were gently vortexed and incubated in the dark for 10-15 minutes at room temperature. Immediately after this incubation period 2 mL of PBS solution was added, followed by a 4-5 seconds vortex. Immediately after this, cells were centrifuged, discarding the supernatant and the cell pellet resuspended in 200 µL of the same solution. To the cell suspension, a rabbit anti-mouse immunoglobulins monoclonal antibody was added at a 1/100 final dilution and a second incubation at room temperature in the dark for 10-15 minutes was performed. Once this incubation was finished 2 mL of an amonium chloride lysing solution was added, followed by a 4-5 seconds vortex. Afterwards, cells were incubated for another 10 minutes in the dark (room temperature). Afterwards samples were centrifuged at 300g for 5 minutes (4°C). The supernatant was discarded and the cell pellet free of red blood cells was resuspended by vigorous vortexing (1-2 seconds). In each tube 1 mL of a phosphate buffer saline solution (PBS) containing 1 g/L of bovine serum albumine (Sigma Chemicals, St Louis, MO, USA) was added. Another centrifugation (300g, 5 minutes), was performed and cells were finally resuspended in 1 mL of PBS solution containing 0.5% tween-20 (Sigma), 100mg/mL de RNAse (Sigma Chemicals) and 5µg/mL of propidium iodide (Sigma Chemicals). Cells were incubated for 15 minutes at room temperature in this solution and were stored at 4°C in the dark until analyzed in the flow cytometer.

### 3.- Data acquisition and analysis.

Measurements were performed on a FACSort (Becton/Dickinson) flow cytometer equipped with an argon ion laser tuned at 488nm and 15 m watts. The instrument was calibrated using the DNA-QC reagent (Becton/Dickinson). Fluorescence compensation between FITC and propidium iodide was performed using CALIBRITE beads (Becton/Dickinson) and chicken red cells stained with propidium iodide.

Data analysis was performed in two steps. First, we selected the cells of interest (FITC positive ) corresponding to plasma cells and excluding not only the remaining cells but also cell debris and cell aggregates. For that purpose the PAINT-A-GATE PLUS (Becton/Dickinson) software program was used. Afterwards the RFIT mathematical model included in the CELLFIT software program (Becton/Dickinson) was used for the analysis of DNA histograms in order to calculate the proportion of cells from the selected plasma cell population in the G0/G1, S y G2/M cell cycle phases.

## Claims

1. Procedure for the analysis of the cell cycle of tumor cell subpopulations present in heterogeneous cell samples, **characterized by** the following steps:
a) sequential incubation of the sample with a pool of the CD38, and CD138 or a pool of the CD19 and CD22 and CD20 and CD37 monoclonal antibodies stained with the same fluorochrome and all designed for the specific and sensitive identification of the cells of interest, and with a nucleic acid specific fluorochrome.
b) to measure by flow cytometry the fluorescence emissions of the two fluorochromes;
c) to analyze the results obtained using multiparametric analysis for the identification of the cell subpopulation to be studied and to analyze its distribution throughout the G0/G1, S y G2/M cell cycle phases.

2. Procedure as in claim 1, **characterized by** the fact that in step (a) normal and pathological samples obtained in vivo, stored or treated in vitro are used.

3. Procedure as in claim 1 **characterized by** the fact that the monoclonal antibodies used and their number may vary depending on the exact tumor cell type to be identified, its phenotype or the type of sample.

4. Procedure as in claim 1 **characterized by** the fact that any combination of fluorochromes can be used if they are technically compatible.

5. Procedure as in claims 1 to 4 **characterized by** the fact that more than one pool of monoclonal antibodies directed aginst more than one different cell subpopulation can be simultaneously used, each of the pools of monoclonal antibodies being stained with a different fluorochrome.

6. Procedure as in claims 1 to 5 in which apart from the staining of the cell subpopulation to be studied and the nucleic acids, other markers including oncoproteins, cell cycle related proteins, cell opoptosis, activation or differentiation markers are identified.

7. Procedure as in claims 1 to 6 in which the identification of cells is performed based on the presence of staining for the pool of monoclonal antibodies used or by the intensity of positivity obtained for them.

## Patentansprüche

1. Verfahren für die Analyse des Zellzyklus von in heterogenen Zellproben vorhandenen Tumorzellsubpopulationen, **gekennzeichnet durch** die folgenden Schritte:
a) sequentielle Inkubation der Probe mit einem Pool von CD38 und CD138 oder einem Pool von CD19 und CD22 und CD20 und CD37 monoklonalen Antikörpern gefärbt mit dem gleichen Fluorochrom, und wobei alle für die spezifische und empfindliche Identifizierung der interessierenden Zellen ausgelegt sind, und mit einem nukleinsäurespezifischen Fluorchrom;
b) Messung der Fluoreszenzemissionen der zwei Fluorochrome **durch** Flusszytometrie;
c) Analyse der erhaltenen Ergebnisse unter Verwendung einer Multiparameteranalyse für den Nachweis der zu untersuchenden Zellsubpopulation, und Analyse ihrer Verteilung über die G0/G1-, S- und G2/M-Zellzyklusphasen.

2. Verfahren nach Anspruch 1, **dadurch gekennzeichnet, dass** in Schritt (a) normale und pathologische *in vivo* erhaltene, *in vitro* gelagerte oder behandelte Proben verwendet werden.

3. Verfahren nach Anspruch 1, **dadurch gekennzeichnet, dass** die verwendeten monoklonalen Antikörper und ihre Anzahl in Abhängigkeit von dem zu identifizierenden genauen Tumorzelltyp, seinem Phänotyp oder der Probenart variieren.

4. Verfahren nach Anspruch 1, **dadurch gekennzeichnet, dass** jede Kombination von Fluorochromen verwendet werden kann, wenn sie technisch kompatibel sind.

5. Verfahren nach einem der Ansprüche 1 bis 4, **dadurch gekennzeichnet, dass** gleichzeitig mehr als ein Pool monoklonaler Antikörper gerichtet gegen mehr als eine unterschiedliche Zellpopulation verwendet werden kann, wobei jeder der Pools der monoklonalen Antikörper mit einem unterschiedlichen Fluorochrom gefärbt wird.

6. Verfahren nach einem der Ansprüche 1 bis 5, wobei neben der Färbung der zu untersuchenden Zellsubpopulation und der Nukleinsäuren andere Marker einschließlich Onkoproteinen, mit dem Zellzyklus verbundene Proteine, Zellapoptose, Aktivierungs- oder Differenzierungsmarker nachgewiesen werden.

7. Verfahren nach einem der Ansprüche 1 bis 6, wobei der Nachweis der Zellen auf der Grundlage der Anwesenheit der Färbung des Pools der verwendeten monoklonalen Antikörper oder durch die Intensität der für sie erhaltenen Positivität durchgeführt wird.

## Revendications

1. Procédé d'analyse du cycle cellulaire de sous-populations de cellules tumorales présentes dans des échantillons de cellules hétérogènes, **caractérisé par** les étapes suivantes consistant à :
a) incuber l'échantillon en séquence avec un groupe d'anticorps monoclonaux CD38 et CD138 ou un groupe d'anticorps monoclonaux CD19 et CD22 et CD20 et CD37 colorés par le même fluorochrome et tous destinés à l'identification spécifique et sensible des cellules d'intérêt, et par un fluorochrome spécifique à l'acide nucléique ;
b) mesurer par cytométrie de flux les émissions de fluorescence des deux fluorochromes ;
c) analyser les résultats obtenus à l'aide de l'analyse multiparamétrique pour l'identification de la sous-population de cellules à étudier et pour analyser sa répartition dans les phases du cycle cellulaire G0/G1, S et G2/M.

2. Procédé selon la revendication 1, **caractérisé par le fait qu'**à l'étape (a) sont utilisés des échantillons normaux et pathologiques obtenus in vivo, stockés ou traités.

3. Procédé selon la revendication 1, **caractérisé par le fait que** les anticorps monoclonaux utilisés et leur nombre peuvent varier selon le type exact de cellules tumorales à identifier, leur phénotype ou le type d'échantillon.

4. Procédé selon la revendication 1, **caractérisé par le fait que** toute combinaison de fluorochromes peut être utilisée s'ils sont techniquement compatibles.

5. Procédé selon les revendications 1 à 4, **caractérisé par le fait que** plus d'un groupe d'anticorps monoclonaux destinés à plus d'une sous-population de cellules différente peuvent être utilisés simultanément, chacun des groupes d'anticorps monoclonaux étant coloré par un fluorochrome différent.

6. Procédé selon les revendications 1 à 5, dans lequel, outre la coloration de la sous-population de cellules à étudier et des acides nucléiques, d'autres marqueurs comportant des oncoprotéines, des protéines relatives au cycle cellulaires, à l'apoptose de cellules, à l'activation ou des marqueurs de différentiation sont identifiés.

7. Procédé selon les revendications 1 à 6, dans lequel l'identification de cellules s'effectue sur base de la présence de la coloration pour le groupe d'anticorps monoclonaux utilisé ou par l'intensité de la positivité obtenue pour ceux-ci.
